(19) Europäisches Patentamt
European Patent Office
Office européen des brevets

(11) Veröffentlichungsnummer: **0 657 435 A2**

(12) **EUROPÄISCHE PATENTANMELDUNG**

(21) Anmeldenummer: **94115937.8**

(22) Anmeldetag: **10.10.94**

(51) Int. Cl.[6]: **C07D 213/64**, A61K 31/44, C07D 407/10, C07D 413/10, C07D 419/10

(30) Priorität: **22.10.93 DE 4336051**

(43) Veröffentlichungstag der Anmeldung: **14.06.95 Patentblatt 95/24**

(84) Benannte Vertragsstaaten: **AT BE CH DE DK ES FR GB GR IE IT LI LU NL PT SE**

(71) Anmelder: **MERCK PATENT GmbH**
**Frankfurter Strasse 250**
**D-64293 Darmstadt (DE)**

(72) Erfinder: **Osswald, Mathias, Dr.**
**Katzenelnbogenweg 1**
**D-64673 Zwingenberg (DE)**
Erfinder: **Mederski, Werner, Dr.**
**Am Ohlenberg 29**
**D-64390 Erzhausen (DE)**
Erfinder: **Dorsch, Dieter, Dr.**
**Königsberger Strasse 17A**
**D-64372 Ober-Ramstadt (DE)**
Erfinder: **Schelling, Pierre, Prof. Dr.**
**Bordenbergweg 17**
**D-64367 Mühltal (DE)**
Erfinder: **Beier, Norbert, Dr.**
**Georgenhäuser-Strasse 19**
**D-64354 Reinheim 5 (DE)**
Erfinder: **Minck, Klaus-Otto, Dr.**
**Büchestrasse 8**
**D-64372 Ober-Ramstadt (DE)**
Erfinder: **Lues, Ingeborg, Dr.**
**Katharinenstrasse 2**
**D-64297 Darmstadt (DE)**

(54) **1,2-Dihydro-2-oxo-pyridine.**

(57) Neue 1,2-Dihydro-2-oxopyridine der Formel I

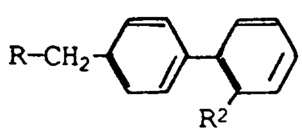

worin
R

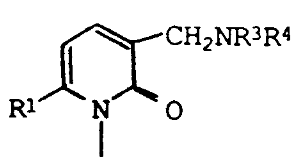

,

bedeutet und $R^1$, $R^2$, $R^3$ und $R^4$ die in Patentanspruch 1 angegebenen Bedeutungen haben, sowie deren Salze zeigen Angiotensin II-antagonistische Eigenschaften und können zur Behandlung von Hypertension, Aldosteronismus, Herzinsuffizienz und erhöhtem Augeninnendruck sowie von Störungen des Zentralnervensystems

EP 0 657 435 A2

verwendet werden.

Die Erfindung betrifft neue 1,2-Dihydro-2-oxopyridine der Formel I

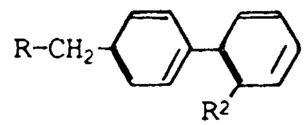

worin

R

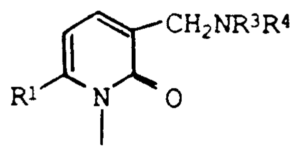

,

| | |
|---|---|
| $R^1$ | H, A, $C_2$-$C_6$-Alkenyl oder $C_2$-$C_6$-Alkinyl, |
| $R^2$ | $-SO_2NH-COOR^5$, $-SO_2NH-COR^5$, $-SO_2NH-SO_2R^5$, $-SO_2NH-CONR^5R^6$, $-C(NH_2)=NOH$, 4,5-Dihydro-5-oxo-1,2,4-oxadiazol-3-yl, 4,5-Dihydro-5-thioxo-1,2,4-oxadiazol-3-yl, 2-Oxo-3H-1,2,3,5-oxathiadiazol-4-yl, 2,2-Dioxo-3H-1,2,3,5-oxathiadiazol-4-yl, 2,3-Dihydro-3-oxo-1,2,4-oxadiazol-5-yl, 2,5-Dihydro-2,5-dioxo-1H-imidazol-4-yl, 4,5-Dihydro-5-oxo-1H-1,2,4-triazol-3-yl, 4,5-Dihydro-5-thioxo-1H-1,2,4-triazol-3-yl, 2,3-Dihydro-2-oxo-1,3,4-thiadiazol-5-yl oder 4,5-Dihydro-5-oxo-1,2,4-thiadiazol-3-yl, |
| $R^3$ | H, A, $C_3$-$C_8$-Cycloalkyl-$C_nH_{2n}$- oder Ar-$C_nH_{2n}$-, |
| $R^4$ | A-CO-, Ar'-CO-, Ar'-$C_mH_{2m}$-CO-, $R^9$-$SO_2$- oder Ar'-$SO_2$-, |
| $R^5$ und $R^6$ | jeweils H, eine $C_1$-$C_6$-Alkylgruppe, worin auch eine $CH_2$-Gruppe durch O oder S ersetzt sein kann oder eine zusätzliche C-C-Doppelbindung enthalten sein kann und welche zusätzlich durch OH, $OR^7$, Ar, Het, $NR^7R^8$, $NR^7$-$COOR^8$, $NR^7$-COO-$C_tH_{2t}$-Ar, $NR^7$-COO-$C_tH_{2t}$-Het und/oder $COOR^7$ substituiert sein kann, $C_3$-$C_8$-Cycloalkyl, Ar oder Het, |
| $R^7$ und $R^8$ | jeweils A, $C_2$-$C_6$-Alkenyl, $C_2$-$C_6$-Alkinyl, $C_3$-$C_8$-Cycloalkyl-$C_kH_{2k}$- oder $C_1$-$C_6$-Alkyl, worin eine $CH_2$-Gruppe durch O oder S ersetzt ist, |
| $R^9$ | $C_1$-$C_6$-Alkyl, worin auch ein oder mehrere H-Atom(e) durch F ersetzt sein können, |
| A | $C_1$-$C_6$-Alkyl, |
| Ar und Ar' | jeweils eine unsubstituierte oder eine durch $R^9$, OH, $OR^9$, COOH, COOA, CN, $NO_2$, $NH_2$, NHA, $N(A)_2$, $NHCOR^9$, NHCOOA, $NHSO_2R^9$, Hal und/oder 1H-5-Tetrazolyl mono- oder disubstituierte Phenylgruppe, |
| Het | einen fünf- oder sechsgliedrigen heteroaromatischen Rest mit 1 bis 3 N-, O- und/oder S-Atomen, der auch mit einem Benzol- oder Pyridinring kondensiert sein kann, |
| Hal | F, Cl, Br oder I, |
| k | 0, 1, 2, 3 oder 4, |
| m und n | jeweils 1, 2 oder 3 und |
| t | 1, 2, 3 oder 4 bedeuten, |

sowie ihre Salze.

Ähnliche Verbindungen sind aus der EP-A2-0530702 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher als Arzneimittelwirkstoffe zur Prophylaxe und/oder Therapie von Herz-, Kreislauf- und Gefäßkrankheiten, vor allem zur Behandlung der angiotensin II-abhängigen Hypertension, des Aldosteronismus, der Herzinsuffizienz und des erhöhten Augeninnendrucks sowie von Störungen des

Zentralnervensystems eingesetzt werden, ferner von Hypertrophie und Hyperplasie der Blutgefäße und des Herzens, Angina pectoris, Herzinfarkt, Schlaganfall, Restenosen nach Angioplastie oder By-pass-Operationen, von ischämischen peripheren Durchblutungsstörungen, Arteriosklerose, Glaukomen, macularer Degeneration, Hyperurikämie, Nierenfunktionsstörungen, z.B. Nierenversagen, Nephropathia diabetica, Retinopathia diabetica, Psoriasis, von gastrointestinalen Erkrankungen, Blasenerkrankungen, Lungenödemen, chronischer Bronchitis, angiotensin II-vermittelten Störungen in weiblichen Fortpflanzungsorganen, Wahrnehmungsstörungen, z.B. Demenz, Amnesie, Gedächtnisfunktionsstörungen, Angstzuständen, Depression, Epilepsie, des Parkinson-Syndroms und/oder der Bulimie.

Diese Wirkungen können nach üblichen in vitro- oder in vivo-Methoden ermittelt werden, wie sie z.B. in der US-PS 4 880 804, der US-PS 5 036 048 und der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Gegenstand der Erfindung sind die Verbindungen der Formel I und ihre Salze sowie ein Verfahren zur Herstellung dieser Verbindungen sowie ihrer Salze, dadurch gekennzeichnet, daß man

(a) eine Verbindung der Formel II

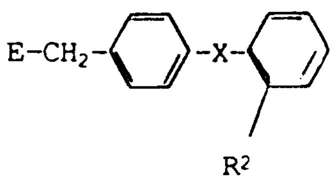

II

worin

E Cl, Br, I oder eine freie oder reaktionsfähig funktionell abgewandelte OH-Gruppe bedeutet und

$R^2$ die in Anspruch 1 angegebene Bedeutung hat,

mit einer Verbindung der Formel III

H-R III

worin

R die in Anspruch 1 angegebene Bedeutung hat,

umsetzt

oder

(b) eine Verbindung der Formel I aus einem ihrer funktionellen Derivate durch Behandeln mit einem solvolysierenden oder hydrogenolysierenden Mittel in Freiheit setzt,

(c) zur Herstellung einer Verbindung der Formel I, worin $R^2$ $-C(NH_2)=NOH$ bedeutet, eine Verbindung, die der Formel I entspricht, aber an Stelle des Restes $R^2$ eine CN-Gruppe trägt, mit Hydroxylamin umsetzt oder

(d) zur Herstellung einer Verbindung der Formel I, worin $R^2$ $-SO_2NH-COOR^5$, $-SO_2NH-COR^5$, $-SO_2NH-SO_2R^5$ oder $-SO_2NH-CONR^5R^6$ bedeutet, eine Verbindung, die der Formel I entspricht, aber an Stelle des Restes $R^2$ eine $-SO_2NH_2$-Gruppe trägt, mit einer Verbindung der Formel $E-COOR^5$, $E-COR^5$, $E-SO_2R^5$, $E-CONR^5R^6$ oder $O=C=NR^5$ umsetzt

und/oder daß man in einer Verbindung der Formel I einen oder mehrere Rest(e) R und/oder $R^2$, in einen oder mehrere andere Reste R und/oder $R^2$ umwandelt und/oder eine Base oder Säure der Formel I in eines ihrer Salze umwandelt.

Vor- und nachstehend haben die Reste bzw. Parameter R, $R^1$ bis $R^9$, A, Ar, Ar', Het, Hal, k, m, n, t und E die bei den Formeln I und II angegebenen Bedeutungen, falls nicht ausdrücklich etwas anderes angegeben ist.

In den vorstehenden Formeln hat A 1-6, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl. 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Alkenyl steht vorzugsweise für Vinyl, 1- oder 2-Propenyl, 1-Butenyl, ferner 1-Pentenyl oder 1-Hexenyl. Alkinyl steht vorzugsweise für Ethinyl, 1- oder 2-Propinyl, ferner 1-Butinyl, 1-Pentinyl oder 1-Hexinyl. Falls mehrere Reste A, Alkenyl oder Alkinyl in einer Verbindung der Formel I vorhanden sind, so können sie gleich oder voneinander verschieden sein.

Hal bedeutet vorzugsweise F, Cl oder Br, aber auch J.

R ist ein von 1,2-Dihydro-2-oxopyridin abgeleiteter Rest, genauer 1,2-Dihydro-2-oxo-3-($R^3R^4$N-methyl)-6-$R^1$-pyridin-1-yl.

Ar und Ar' sind jeweils unabhängig voneinander vorzugsweise unsubstituiertes, ferner - wie angegeben - monosubstituiertes Phenyl, im einzelnen bevorzugt Phenyl, o-, m- oder p-Tolyl, o-, m- oder p-Ethylphenyl, o-, m- oder p-Trifluormethylphenyl, o-, m- oder p-Methoxyphenyl, o-, m- oder p-Ethoxyphenyl, o-, m- oder p-(Difluormethoxy)-phenyl, o-, m- oder p-(Trifluormethoxy)-phenyl, o-, m- oder p-Carboxyphenyl, o-, m- oder p-Methoxycarbonylphenyl, o-, m- oder p-Ethoxycarbonylphenyl, o-, m- oder p-Cyanphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Trifluoracetamidophenyl, o-, m- oder p-Methoxycarbonylaminophenyl, o-, m- oder p-Ethoxycarbonylaminophenyl, o-, m- oder p-Methylsulfonamidophenyl, o-, m- oder p-Trifluormethylsulfonamidophenyl, o-, m- oder p-Fluorphenyl, o-, m- oder p-Chlorphenyl, o-, m- oder p-Bromphenyl, o-, m- oder p-(1H-5-Tetrazolyl)-phenyl, ferner bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -4-yl, 2,1,5-Thiadiazol-3- oder -4-yl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2-1,3-oxadiazolyl, 2-, 3-, 4-, 5-, 6-, 7- oder 8-Chinolinyl, 1-, 3-, 4-, 5-, 6-, 7- oder 8-Isochinolinyl, 3-, 4-, 5-, 6-, 7- oder 8-Cinnolinyl, 2-, 4-, 5-, 6-, 7- oder 8-Chinazolinyl, 1H-1-, -2-, -5-, -6- oder -7-Imidazo[4,5-b]pyridyl, 3H-2-, -3-, -5-, -6- oder -7-Imidazo[4,5-b]pyridyl, 1H-1-, -2-, -4-, -6- oder -7-Imidazo[4,5-c]pyridyl, 3H-2-, -3-, -4-, -6- oder -7-Imidazo[4,5-c]pyridyl.

Die Gruppen $-C_kH_{2k}-$, $-C_mH_{2m}-$, $-C_nH_{2n}-$ und $-C_tH_{2t}-$ sind vorzugsweise geradkettig und stehen somit bevorzugt für $-(CH_2)_k-$,$-(CH_2)_m-$, $-(CH_2)_n-$ und $-(CH_2)_t-$, insbesondere für $-CH_2-$, ferner für $-CH_2CH_2-$, $-(CH_2)_3-$, $-(CH_2)_4-$, $-(CH_2)_5-$ oder $-(CH_2)_6-$, aber auch z.B. für $-CH(CH_3)-$, $-CH_2-CH(CH_3)-$ oder $-C(CH_3)_2-$. Der Parameter k kann bevorzugt auch 0 sein, so daß die Gruppe $-C_kH_{2k}-$ fehlt.

Der Rest $R^1$ ist vorzugsweise geradkettig und steht bevorzugt für A, insbesondere Ethyl, Propyl oder Butyl, ferner Methyl, Pentyl oder Hexyl, weiterhin insbesondere für Alkenyl mit vorzugsweise 3-6 C-Atomen, vor allem Allyl oder 1-Propenyl, ferner 1-Butenyl, 1-Pentenyl oder 1-Hexenyl; für Alkinyl mit vorzugsweise 3-6 C-Atomen, vor allem Propargyl oder 1-Propinyl, ferner 1-Butinyl, 1-Pentinyl oder 1-Hexinyl.

Der Rest $R^2$ ist vorzugsweise $-SO_2NH-COOR^5$, insbesondere $-SO_2NH-COOA$; $-SO_2NH-CO-R^5$, insbesondere $-SO_2NH-COAr$ wie $-SO_2NH-COC_6H_5$; $-SO_2NH-CONR^5R^6$, insbesondere $-SO_2NH-CO-NHAr$ wie $-SO_2NH-CO-NHC_6H_5$ oder $-SO_2NH-CO-NHCH_2Het^2$ wie $-SO_2NH-CO-NHCH_2$-(2-pyridyl). Der Rest $R^2$ ist ferner vorzugsweise 4,5-Dihydro-5-oxo-1,2,4-oxadiazol-3-yl, ferner bevorzugt 4,5-Dihydro-5-thioxo-1,2,4-oxadiazol-3-yl, 2-Oxo-3H-1,2,3,5-oxathiadiazol-4-yl, 2,2-Dioxo-3H-1,2,3,5-oxathiadiazol-4-yl oder 4,5-Dihydro-5-oxo-1,2,4-thiadiazol-3-yl.

Der Rest $R^3$ steht bevorzugt für H; A, insbesondere Methyl, ferner Ethyl, Propyl, Isopropyl, Butyl oder Isobutyl; Cyclopropylmethyl.

Der Rest $R^4$ ist vorzugsweise $R^9-SO_2-$.

Der Rest $R^5$ ist vorzugsweise A, insbesondere Methyl, Ethyl, Propyl oder Butyl; Ar, insbesondere Phenyl; Het-alkyl, insbesondere 2-, 3- oder 4-Pyridyl-methyl; oder Cycloalkyl, insbesondere Cyclopropyl.

Der Rest $R^6$ ist vorzugsweise H, ferner A.

Die Reste $R^7$ und $R^8$ sind vorzugsweise jeweils A.

Der Rest $R^9$ steht vorzugsweise für A, insbesondere Methyl, ferner Ethyl,Propyl, Isopropyl, Butyl oder Isobutyl.

Der Parameter k ist vorzugsweise 0 oder 1. Der Parameter m ist vorzugsweise 1 oder 2. Die Parameter n und t sind vorzugsweise 1, ferner bevorzugt 2, 3 oder 4.

Die Verbindungen der Formel I können ein odere mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ic

ausgedrückt werden, die der Formel I entsprechen und worin die nicht näher bezeichneten Reste die bei Formel I angegebenen Bedeutungen haben, worin jedoch:

in Ia $R^1$ A bedeutet;

in Ib $R^2$ 4,5-Dihydro-5-oxo-1,2,4-oxadiazol-3-yl bedeutet;

in Ic $R^1$ A und $R^2$ 4,5-Dihydro-5-oxo-1,2,4-oxadiazol-3-yl bedeuten.

Weiterhin sind bevorzugt:

Verbindungen der Formeln Id sowie Iad bis Icd, die den Verbindungen der Formeln I sowie Ia bis Ic entsprechen, worin jedoch zusätzlich $R^3$ A oder Cyclopropylmethyl bedeutet;

Verbindungen der Formeln Ie, Iae bis Ide, sowie Iade bis Icde, die den Formeln I, Ia bis Id sowie Iad bis Icd entsprechen, worin jedoch zusätzlich $R^4$ A-$SO_2$- bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP-A2-O 430 709 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

(a) Die Verbindungen der Formel I können erhalten werden, indem man Verbindungen der Formel II mit Verbindungen der Formel III umsetzt.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsufonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie $CH_3ONa$ oder K-tert.-Butylat in einem Alkohol wie Methanol oder tert.-Butanol oder mit einem Alkalimetallcarbonat wie $K_2CO_3$ oder mit einem Alkalimetallhydrid wie NaH oder einem Alkalimetallalkoholat in Dimethylformamid (DMF), und dieses dann in einem inerten Lösungsmittel, z.B. einem Amid wie DMF, N-Methylpyrrolidon oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetall-hydrogencarbonate wie $NaHCO_3$ oder $KHCO_3$.

(b) Weiterhin kann man eine Verbindung der Formel I durch Solvolyse (z.B. Hydrolyse) oder Hydrogenolyse aus einem ihrer funktionellen Derivate in Freiheit setzen.

So kann man Carbonsäuren der Formel I, die (mindestens) eine COOH-Gruppe enthalten, erhalten durch Verseifung entsprechender Alkylester, z.B. mit NaOH oder KOH in wässeriger Lösung mit oder ohne Zusatz eines inerten organischen Lösungsmittels wie Methanol, Ethanol, THF oder Dioxan bei Temperaturen zwischen 0 und 100°, oder durch Hydrogenolyse entsprechender Benzylester, z.B. an Pd-Kohle bei Drucken zwischen 1 und 200 bar und bei Temperaturen zwischen 0 und 100° in einem der angegebenen inerten Lösungsmittel. Es ist auch möglich, eine Verbindung, die der Formel I entspricht, aber an Stelle des Restes $R^2$ eine 5-Trichlormethyl-1,2,4-oxadiazol-3-yl-Gruppe enthält (erhältlich durch Reaktion eines Carboxamid-oxims der Formel I, $R^2$ = $-C(NH_2)=NOH$, mit Trichloressigsäure-anhydrid) alkalisch zu spalten, z.B. mit NaOH in Wasser/Dioxan bei 0-10°, wobei man die entsprechende Verbindung I, $R^2$ = 4,5-Dihydro-5-oxo-1,2,4-oxadiazol-3-yl, erhält.

(c) Umsetzung eines Nitrils, das der Formel I entspricht, aber an Stelle des Restes $R^2$ eine CN-Gruppe enthält, mit Hydroxylamin liefert das entsprechende Carboxamid-oxim I, $R^2$ = $-C(NH_2)=NOH$. Bei dieser Reaktion arbeitet man zweckmäßig in einem inerten Lösungsmittel wie THF bei Temperaturen zwischen 20 und 100°.

(d) Verbindungen der Formel I, worin $R^2$ $-SO_2NH-COOR^5$, $-SO_2NH-COR^5$, $-SO_2NH-SO_2R^5$ oder $-SO_2NH-CONR^5R^6$ bedeutet, können durch N-Acylierung von Verbindungen erhalten werden, die der Formel I entsprechen, aber an Stelle eines Restes $R^2$ eine $-SO_2NH_2$-Gruppe enthalten. Als Acylierungsmittel eignen sich z.B. Verbindungen der Formeln $E-COOR^5$, z.B. Chlorameisensäure-methyl- und -ethylester; $E-COR^5$, z.B. Acetylchlorid, Cyclopropancarbonylchlorid, oder Benzoylchlorid; $E-SO_2R^5$, z.B. Methansulfonylchlorid, p-Toluolsulfonylchlorid; $E-CO-NR^5R^6$, z.B. Diphenylcarbamoylchlorid; $O=C=NR^5$, z.B. Phenylisocyanat.

Die Umsetzung wird in der Regel in Gegenwart einer Base, vorzugsweise eines tertiären Amins, z.B. Triethylamin, Pyridin, 4-Dimethylaminopyridin vorgenommen, zweckmäßig bei Temperaturen zwischen 0 und 100°. Ein Überschuß des Amins kann auch als Lösungsmittel dienen. Die Reaktion mit Isocyanaten der Formel $O=C=NR^5$ zu den entsprechenden Sulfonylharnstoffen wird bevorzugt bei Temperaturen

zwischen 50 und 100° vorgenommen, wobei ein Überschuß des Isocyanats als Lösungsmittel dient.

Die Ausgangsstoffe, insbesondere diejenigen der Formel II, sind teilweise bekannt. Falls sie nicht bekannt sind, können sie nach bekannten Methoden in Analogie zu bekannten Stoffen hergestellt werden.

Es ist ferner möglich, eine Verbindung der Formel I in eine andere Verbindung der Formel I umzuwandeln, indem man einen oder mehrere der Reste R und/oder $R^2$ in andere Reste R und/oder $R^2$ umwandelt, z.B. indem man eine Verbindung der Formel I ($R^3$ = H) mit einer Verbindung der Formel E-$R^3$ (worin $R^3$ von H verschieden ist) umsetzt oder Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Nitrilgruppen zu COOH-Gruppen hydrolysiert und/oder eine Carbonsäuregruppe verestert, z.B. durch Umsetzung mit einem Alkohol der Formel A-OH und/oder eine Carbamidoximgruppe durch Umsetzung (a) mit 1,1'-Carbonyldiimidazol oder einem Chlorameisenäurealkylester in eine 4,5-Dihydro-5-oxo-1,2,4-oxadiazol-3-yl-gruppe, (b) mit 1,1'-Thiocarbonyl-diimidazol in eine 4,5-Dihydro-5-oxo-1,2,4-thiadiazol-3-yl-gruppe, (c) mit $SOCl_2$ in eine 2-Oxo-3H-1,2,3,5-oxathiadiazol-4-yl-gruppe oder (d) mit $SO_2Cl_2$ in eine 2,2-Dioxo-3H-1,2,3,5-oxathiadiazol-4-yl-gruppe umwandelt und/oder eine -$SO_2$NH-COO$R^5$-Gruppe durch Amidierung mit einer Verbindung der Formel HN$R^5R^6$ in eine -$SO_2$NH-CO-N$R^5R^6$-Gruppe umwandelt.

So kann man Verbindungen der Formel I ($R^3$ = H) durch Reaktion mit Verbindungen der Formel E-$R^3$ (worin $R^3$ von H verschieden ist) alkylieren. Man arbeitet dabei bevorzugt in einem inerten Lösungsmittel, z.B. einem Säureamid wie DMF, N-Methylpyrrolidon, 1,3-Dimethyl-2-oxo-hexahydropyrimidin oder Phosphorsäure-hexamethyl-triamid, einem Alkohol wie Methanol oder tert.-Butanol, einem Ether wie THF oder einem halogenierten Kohlenwasserstoff wie Dichlormethan oder Gemischen davon als Lösungsmittel und/oder in Gegenwart eines Alkalimetallalkoholats wie Natriummethylat oder Kalium-tert.-butylat, eines Alkalimetallhydrids wie Natrium- oder Kaliumhydrid, eines Alkalimetallcarbonats wie Natrium- oder Kaliumcarbonat, eines Alkalimetallbicarbonats wie Natrium- oder Kaliumbicarbonat oder eines tertiären Amins wie Triethylamin oder Ethyldiisopropylamin bei Temperaturen zwischen etwa -30 und 200, vorzugsweise zwischen 20 und 60°.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z.B. eine Verbindung der Formel I, die eine NHCO$R^9$- oder eine COOA-Gruppe enthält, in die entsprechende Verbindung der Formel I umgewandelt werden, die stattdessen eine $NH_2$- oder eine HOOC-Gruppe enthält. COOA-Gruppen können z.B. mit NaOH oder KOH in Wasser, Wasser-THF oder Wasser-Dioxan bei Temperaturen zwischen 0 und 100° verseift werden.

Die oben genannten Umwandlungen einer Carbamidoximgruppe (I, $R^2$ = -C($NH_2$)=NOH) in verschiedene oben genannte heterocyclische Reste erfolgt bevorzugt in Gegenwart eines inerten Lösungsmittels, z.B. eines Ethers wie THF, eines Kohlenwasserstoffs wie Toluol, eines Amids wie DMF, eines halogenierten Kohlenwasserstoffs wie Dichlormethan oder einer Base wie Pyridin oder eines Gemisches zweier dieser Lösungsmittel bei Temperaturen zwischen 0 und 100°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure; 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z.B. Pikrate, können zur Isolierung und/oder Aufreinigung der Verbindung der Formel I verwendet werden.

Andererseits können Verbindungen der Formel I, die COOH- oder z.B. 1,2,4-Oxadiazolgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.

Die Kaliumsalze der 1,2,4-Oxadiazolderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind spezielle Lacktabletten und Kapseln mit magensaftresistenten Überzügen bzw. Kapselhüllen. Zur rektalen Anwendung dienen-Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, z.B. Captopril oder Enalapril, insbesondere aber in Analogie zu den in der US-PS 4 880 804 beschriebenen Verbindungen verabreicht, vorzugsweise in Dosierungen zwischen etwa 1 mg und 1 g, insbesondere zwichen 50 und 500 mg pro Dosierungseinheit. Die tägliche Dosierung liegt vorzugsweise zwischen etwa 0,1 und 50 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. FAB = massenspektroskopisch nach der "fast atom bombardment"-Methode erhaltener Molionenpeak ($M^+ + 1$).

Beispiel 1

Eine Lösung von 2,72 g 1,2-Dihydro-2-oxo-3-N-methyl-N-methylsulfonyl-aminomethyl-6-butyl-pyridin (FAB 273; erhältlich durch Reaktion von 1,2-Dihydro-2-oxo-6-butyl-pyridin-3-carboxaldehyd mit Methylamin/$H_2$ zu 1,2-Dihydro-2-oxo-3-methylaminomethyl-6-butyl-pyridin und anschließende Umsetzung mit Methansulfonylchlorid) in 30 ml DMF wird mit 1,12 g K-tert.-Butylat versetzt und 1 Std. bei 20° gerührt. Anschließend tropft man bei 0° unter Rühren eine Lösung von 3,31 g 4-Brommethyl-2'-(4,5-dihydro-5-oxo-1,2,4-oxadiazol-3-yl)-biphenyl (erhältlich durch Reaktion von 4-Methyl-2'-cyan-biphenyl mit Hydroxylamin zu 4-Methyl-2'-(amino-oximinomethyl)-biphenyl, Umsetzung mit Ethyl-chlorformiat zu 4-Methyl-2'-(4,5-dihydro-5-oxo-1,2,4-oxadiazol-3-yl)-biphenyl und Bromierung) in 35 ml DMF hinzu. Man rührt 16 Std. bei 20°, dampft ein, arbeitet wie üblich auf und erhält 1,2-Dihydro-1-(2'-(4,5-dihydro-5-oxo-1,2,4-oxadiazol-3-yl)-biphenylyl-4-methyl)-2-oxo-3-N-methyl-N-methylsulfonyl-aminomethyl-6-butyl-pyridin ("Ix"), F. 207°. K-Salz, Rf 0,27 an Kieselgel (Ethylacetat).

Beispiel 2

Man tropft unter Eiskühlung 1,5 ml 1N NaOH zur einer Lösung von 1 g 1,2-Dihydro-1-(2'-(5-trichlormethyl-1,2,4-oxadiazol-3-yl)-biphenylyl-4-methyl)-2-oxo-3-N-methyl-N-methylsulfonylaminomethyl-6-butyl-pyridin (erhältlich aus 3-(4'-Brommethyl-2-biphenylyl)-5-trichlormethyl-1,2,4-oxadiazol (EP-A2-520423, Beispiel 22 c) und 1,2-Dihydro-2-oxo-3-N-methyl-N-methylsulfonyl-aminomethyl-6-butyl-pyridin analog Beispiel 1) in einem Gemisch von 8 ml Dioxan und 2 ml Wasser, rührt 30 Min. unter Eiskühlung, arbeitet wie üblich auf (verdünnte Salzsäure/Ethylacetat) und erhält "Ix", F. 207°.

## Beispiel 3

a) Eine Lösung von 7,1 g Hydroxylammoniumchlorid in 30 ml DMSO wird mit 14,4 ml Triethylamin in 40 ml THF versetzt. Man filtriert, dampft das Filtrat ein und versetzt es mit 4,63 g 1,2-Dihydro-1-(2'-cyan-biphenylyl-4-methyl)-2-oxo-3-N-methyl-N-methylsulfonyl-aminomethyl-6-butyl-pyridin (Rf 0,35 (Petrolether/Ethylacetat 4:6); erhältlich durch Reaktion von 1,2-Dihydro-2-oxo-3-N-methyl-N-methylsulfonyl-aminomethyl-6-butyl-pyridin mit 4'-Brommethyl-2-cyan-biphenyl) sowie weiteren 3,5 ml Triethylamin. Die Lösung wird 16 Std. bei 75° gerührt. Man kühlt ab, gießt in Wasser, filtriert das ausgefallene 1,2-Dihydro-1-(2'-(amino-oximino-methyl)-biphenylyl-4-methyl)-2-oxo-3-N-methyl-N-methylsulfonyl-aminomethyl-6-butyl-pyridin ("A") ab und trocknet.

b) Eine Suspension von 4,96 g "A" (roh)) in 32 ml THF wird mit 2,4 g 1,1'-Carbonyldiimidazol versetzt und 7 Std. unter Rühren gekocht. Man dampft ein, arbeitet wie üblich auf (Ethylacetat/verdünnte $H_2SO_4$) und erhält "Ix", F. 207°.

Analog a) erhält man aus den nachstehenden 1,2-Dihydro-1-(2'-cyan-biphenylyl-4-methyl)-2-oxo-3-N-$R^3$-N-methylsulfonyl-aminomethyl-6-butyl-pyridinen (erhältlich durch Reaktion von 1,2-Dihydro-2-oxo-6-butyl-pyridin-3-carboxaldehyd mit Aminen der Formel $R^3$-$NH_2$/$H_2$ zu 1,2-Dihydro-2-oxo-3-N-$R^3$-aminomethyl-6-butyl-pyridinen, Umsetzung mit Methansulfonylchlorid/Pyridin zu 1,2-Dihydro-2-oxo-3-N-$R^3$-N-methylsulfonyl-aminomethyl-6-butyl-pyridinen sowie Reaktion mit 4'-Brommethyl-2-cyan-biphenyl):

-3-N-ethyl-
-3-N-propyl-
-3-N-isopropyl-
-3-N-butyl-
-3-N-isobutyl-
-3-N-cyclopropylmethyl-

mit Hydroxylammoniumchlorid/Triethylamin die nachstehenden 1,2-Dihydro-1-(2'-(amino-oximino-methyl)-biphenylyl-4-methyl)-2-oxo-3-N-$R^3$-N-methylsulfonyl-aminomethyl-6-butylpyridine:

-3-N-ethyl-
-3-N-propyl-
-3-N-isopropyl-
-3-N-butyl-
-3-N-isobutyl-
-3-N-cyclopropylmethyl-

und daraus analog b) mit 1,1'-Carbonyldiimidazol die nachstehenden 1,2-Dihydro-1-(2'-(4,5-dihydro-5-oxo-1,2,4-oxadiazol-3-yl)-biphenylyl-4-methyl)-2-oxo-3-N-$R^3$-N-methylsulfonylaminomethyl-6-butyl-pyridine:

-3-N-ethyl-
-3-N-propyl-
-3-N-isopropyl-
-3-N-butyl-
-3-N-isobutyl-
-3-N-cyclopropylmethyl-, K-Salz, F. 203°

## Beispiel 4

a) Zu einer Lösung von 527 mg 1,2-Dihydro-1-(2'-aminosulfonyl-biphenylyl-4-methyl)-2-oxo-3-N-methyl-N-methylsulfonyl-aminomethyl-6-butyl-pyridin ("B"; erhältlich durch Reaktion von 1,2-Dihdro-2-oxo-3-N-methyl-N-methylsulfonyl-aminomethyl-6-butyl-pyridin mit 4'-Brommethyl-biphenyl-2-sulfonsäure-(N-tert.-butylamid) zu 1,2-Dihydro-1-(2'-N-tert.-butyl-aminosulfonyl-biphenylyl-4-methyl)-2-oxo-3-N-methyl-N-methylsulfonyl-aminomethyl-6-butylpyridinund Abspaltung der tert.-Butylgruppe mit $CF_3COOH$/Anisol) und 360 mg 4-Dimethylaminopyridin in 12 ml Pyridin gibt man 550 mg Ethylchlorformiat, rührt das Gemisch 48 Std. bei 20°, gibt 8 ml Methanol hinzu und arbeitet wie üblich auf. Man erhält 1,2-Dihydro-1-(2'-(N-ethoxycarbonyl-aminosulfonyl)-biphenylyl-4-methyl)-2-oxo-3-N-methyl-N-methylsulfonyl-aminomethyl-6-butyl-pyridin.

Analog erhält man aus "B" und Butylchlorformiat das 1,2-Dihydro-1-(2'-(N-butoxycarbonyl-aminosulfonyl)-biphenylyl-4-methyl)-2-oxo-3-N-methyl-N-methylsulfonyl-aminomethyl-6-butylpyridin.

Analog erhält man mit Acetylchlorid, Propionylchlorid, Butyrylchlorid, Cyclopropylcarbonylchlorid, Cyclopropylacetylchlorid, Cyclopentylcarbonylchlorid, Benzoylchlorid, p-Methoxybenzoylchlorid, p-Chlorbenzoylchlorid oder 2-Thienylcarbonylchlorid an Stelle von Ethylchlorformiat die nachstehenden 1,2-Dihydro-1-(2'-$R^2$-biphenylyl-4-methyl)-2-oxo-3-N-methyl-N-methylsulfonyl-aminomethyl-6-butyl-pyridine:

-3-(2'-(acetyl-aminosulfonyl)-biphenylyl-4-methyl)-
-3-(2'-(propionyl-aminosulfonyl)-biphenylyl-4-methyl)-
-3-(2'-(butyryl-aminosulfonyl)-biphenylyl-4-methyl)-
-3-(2'-(cyclopropylcarbonyl-aminosulfonyl)-biphenylyl-4-methyl)-
-3-(2'-(cyclopropylacetyl-aminosulfonyl)-biphenylyl-4-methyl)-
-3-(2'-(cyclopentylcarbonyl-aminosulfonyl)-biphenylyl-4-methyl)-
-3-(2'-(benzoyl-aminosulfonyl)-biphenylyl-4-methyl)-
-3-(2'-(p-methoxybenzoyl-aminosulfonyl)-biphenylyl-4-methyl)-
-3-(2'-(p-chlorbenzoyl-aminosulfonyl)-biphenylyl-4-methyl)-
-3-(2'-(2-thienylcarbonyl-aminosulfonyl)-biphenylyl-4-methyl)-.

b) Eine Lösung von 598 mg der nach (a) erhaltenen Ethoxycarbonylverbindung und 108 mg 2-Aminomethylpyridin in 30 ml Toluol wird 2 Std. gekocht, abgekühlt und wie üblich aufgearbeitet. Man erhält 1,2-Dihydro-1-(2'-N-(N-2-pyridylmethyl-carbamoyl)-aminosulfonyl-biphenylyl-4-methyl)-2-oxo-3-N-methyl-N-methylsulfonyl-aminomethyl-6-butylpyridin.

Beispiel 5

Ein Gemisch von 527 mg "B" und 7 ml Phenylisocyanat wird 4 Std. auf 80° erhitzt. Man kühlt ab, destilliert das überschüssige Phenylisocyanat ab und erhält nach chromatographischer Aufreinigung 1,2-Dihydro-1-(2'-N-(N-phenylcarbamoyl)-aminosulfonyl-biphenylyl-4-methyl)-2-oxo-3-N-methyl-N-methylsulfonyl-aminomethyl-6-butyl-pyridin.

Beispiel 6

Man suspendiert 496 mg "A" unter Argon in 7 ml Pyridin und versetzt bei 0° unter Rühren tropfenweise mit einer Lösung von 0,09 ml $SOCl_2$ in 3,5 ml $CH_2Cl_2$. Man rührt noch 2 Std. bei 0°, gießt in 500 ml Wasser, arbeitet wie üblich auf und erhält 1,2-Dihydro-1-(2'-(2-oxo-3H-1,2,3,5-oxathiadiazol-4-yl)-biphenylyl-4-methyl)-2-oxo-3-N-methyl-N-methylsulfonyl-aminomethyl-6-butyl-pyridin.

Beispiel 7

Analog Beispiel 6 erhält man aus "A" und $SO_2Cl_2$ das 1,2-Dihydro-1-(2'-(2,2-dioxo-3H-1,2,3,5-oxathiadiazol-4-yl)-biphenylyl-4-methyl)-2-oxo-3-N-methyl-N-methylsulfonyl-aminomethyl-6-butyl-pyridin.

Beispiel 8

Ein Gemisch von 496 mg "A", 10 ml THF und 180 mg 1,1'-Thiocarbonyldiimidazol wird 30 Min. bei 20° gerührt und eingedampft. Man löst den Rückstand in Ethylacetat, wäscht mit verdünnter Salzsäure, dann mit Wasser, trocknet und dampft erneut ein. Der so erhaltene Rückstand wird in einem Gemisch von 60 ml Chloroform und 12 ml Methanol gelöst. Zu dieser Lösung gibt man 3,5 g Kieselgel, rührt 48 Std. bei 20°, filtriert, dampft ein und reinigt chromatographisch an Kieselgel. Man erhält 1,2-Dihydro-1-(2'-(4,5-dihydro-5-oxo-1,2,4-thiadiazol-3-yl)-biphenylyl-4-methyl)-2-oxo-3-N-methyl-N-methylsulfonyl-aminomethyl-6-butyl-pyridin.

Beispiel 9

Man löst 4,96 g "A" in 50 ml Dichlormethan, gibt 1,15 g Triethylamin und 1,15 g Acetanhydrid hinzu und rührt 2 Std. bei 20°. Man dampft ein, arbeitet wie üblich auf (Ethylacetat/Wasser; Waschen mit $NaHCO_3$-Lösung) und löst das erhaltene rohe Acetylderivat in 30 ml DMF. Man gibt zunächst 4 g $CS_2$, dann innerhalb von 10 Minuten NaH (60 %, in Öl; 1,4 g) hinzu und rührt 2 Std. bei 20°. Nach üblicher Aufarbeitung (pH 3, Eiswasser) erhält man 1,2-Dihydro-1-(2'-(4,5-dihydro-5-thioxo-1,2,4-oxadiazol-3-yl)-biphenylyl-4-methyl)-2-oxo-3-N-methyl-N-methylsulfonyl-aminomethyl-6-butyl-pyridin.

Beispiel 10

a) Analog Beispiel 3a) erhält man aus 1,2-Dihydro-1-(2'-cyan-biphenylyl-4-methyl)-2-oxo-3-N-methyl-N-methylsulfonyl-aminomethyl-6-propyl-pyridin (erhältlich durch Reaktion von 1,2-Dihydro-2-oxo-3-N-methyl-N-methylsulfonylaminomethyl-6-propyl-pyridin mit 4'-Brommethyl-2-cyan-biphenyl) mit Hydroxylammoniumchlorid/Triethylamin das 1,2-Dihydro-1-(2'-(amino-oximino-methyl)-biphenylyl-4-methyl)-2-oxo-3-N-methyl-N-methylsulfonyl-aminomethyl-6-propyl-pyridin.

b) Analog Beispiel 3b) erhält man aus der nach a) hergestellten Verbindung mit 1,1'-Carbonyldiimidazol das 1,2-Dihydro-1-(2'-(4,5-dihydro-5-oxo-1,2,4-oxadiazol-3-yl)-biphenylyl-4-methyl)-2-oxo-3-N-methyl-N-methylsulfonylaminomethyl-6-propyl-pyridin.

Analog a) erhält man aus den nachstehenden 1,2-Dihydro-1-(2'-cyan-biphenylyl-4-methyl)-2-oxo-3-N-$R^3$-N-methylsulfonyl-aminomethyl-6-propyl-pyridinen (erhältlich durch Reaktion von 1,2-Dihydro-2-oxo-6-propyl-pyridin-3-carboxaldehyd mit Aminen der Formel $R^3$-$NH_2$/$H_2$ zu 1,2-Dihydro-2-oxo-3-N-$R^3$-aminomethyl-6-propyl-pyridinen, Umsetzung mit Methansulfonylchlorid/Pyridin zu 1,2-Dihydro-2-oxo-3-N-$R^3$-N-methylsulfonyl-aminomethyl-6-propyl-pyridinen sowie Reaktion mit 4'-Brommethyl-2-cyan-biphenyl):

-3-N-ethyl-
-3-N-propyl-
-3-N-isopropyl-
-3-N-butyl-
-3-N-isobutyl-
-3-N-cyclopropylmethyl-

mit Hydroxylammoniumchlorid/Triethylamin die nachstehenden 1,2-Dihydro-1-(2'-amino-oximino-methyl)-biphenylyl-4-methyl)-2-oxo-3-N-$R^3$-N-methylsulfonyl-aminomethyl-6-propylpyridine:

-3-N-ethyl-
-3-N-propyl-
-3-N-isopropyl-
-3-N-butyl-
-3-N-isobutyl-
-3-N-cyclopropylmethyl-

und daraus analog b) mit 1,1'-Carbonyldiimidazol die nachstehenden 1,2-Dihydro-1-(2'-(4,5-dihydro-5-oxo-1,2,4-oxadiazol-3-yl)-biphenylyl-4-methyl)-2-oxo-3-N-$R^3$-N-methylsulfonylaminomethyl-6-propyl-pyridine:

-3-N-ethyl-
-3-N-propyl-
-3-N-isopropyl-
-3-N-butyl-
-3-N-isobutyl-
-3-N-cyclopropylmethyl-, öl.

Die nachstehenden Beispiele betreffen pharmazeutische Zubereitungen, die Wirkstoffe der Formel I oder ihre Salze enthalten.

Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

Beispiel C: Weichgelatine-Kapseln

Übliche Weichgelatine-Kapseln werden mit einem Gemisch aus jeweils 50 mg Wirkstoff und 250 mg Olivenöl gefüllt.

Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

Beispiel E: Wässerige Suspension für orale Applikation

Eine wässerige Suspension des Wirkstoffs wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

**Patentansprüche**

**1.** 1,2-Dihydro-2-oxopyridine der Formel I

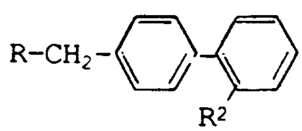

worin

R

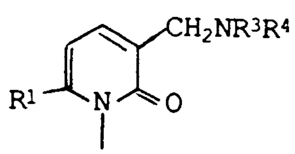

,

$R^1$ H, A, $C_2$-$C_6$-Alkenyl oder $C_2$-$C_6$-Alkinyl,

$R^2$ $-SO_2NH-COOR^5$, $-SO_2NH-COR^5$, $-SO_2NH-SO_2R^5$, $-SO_2NH-CONR^5R^6$, $-C(NH_2)=NOH$,
4,5-Dihydro-5-oxo-1,2,4-oxadiazol-3-yl,
4,5-Dihydro-5-thioxo-1,2,4-oxadiazol-3-yl,
2-Oxo-3H-1,2,3,5-oxathiadiazol-4-yl,
2,2-Dioxo-3H-1,2,3,5-oxathiadiazol-4-yl,
2,3-Dihydro-3-oxo-1,2,4-oxadiazol-5-yl,
2,5-Dihydro-2,5-dioxo-1H-imidazol-4-yl,
4,5-Dihydro-5-oxo-1H-1,2,4-triazol-3-yl,
4,5-Dihydro-5-thioxo-1H-1,2,4-triazol-3-yl,
2,3-Dihydro-2-oxo-1,3,4-thiadiazol-5-yl oder

4,5-Dihydro-5-oxo-1,2,4-thiadiazol-3-yl,

| | |
|---|---|
| $R^3$ | H, A, $C_3$-$C_8$-Cycloalkyl-$C_nH_{2n}$- oder Ar-$C_nH_{2n}$-, |
| $R^4$ | A-CO-, Ar'-CO-, Ar'-$C_mH_{2m}$-CO-, $R^9$-$SO_2$- oder Ar'-$SO_2$-, |
| $R^5$ und $R^6$ | jeweils H, eine $C_1$-$C_6$-Alkylgruppe, worin auch eine $CH_2$-Gruppe durch O oder S ersetzt sein kann oder eine zusätzliche C-C-Doppelbindung enthalten sein kann und welche zusätzlich durch OH, $OR^7$, Ar, Het, $NR^7R^8$, $NR^7$-$COOR^8$, $NR^7$-COO-$C_tH_{2t}$-Ar, $NR^7$-COO-$C_tH_{2t}$-Het und/oder $COOR^7$ substituiert sein kann, $C_3$-$C_8$-Cycloalkyl, Ar oder Het, |
| $R^7$ und $R^8$ | jeweils A, $C_2$-$C_6$-Alkenyl, $C_2$-$C_6$-Alkinyl, $C_3$-$C_8$-Cycloalkyl-$C_kH_{2k}$- oder $C_1$-$C_6$-Alkyl, worin eine $CH_2$-Gruppe durch O oder S ersetzt ist, |
| $R^9$ | $C_1$-$C_6$-Alkyl, worin auch ein oder mehrere H-Atom(e) durch F ersetzt sein können, |
| A | $C_1$-$C_6$-Alkyl, |
| Ar und Ar' | jeweils eine unsubstituierte oder eine durch $R^9$, OH, $OR^9$, COOH, COOA, CN, $NO_2$, $NH_2$, NHA, $N(A)_2$, $NHCOR^9$, NHCOOA, $NHSO_2R^9$, Hal und/oder 1H-5-Tetrazolyl mono- oder disubstituierte Phenylgruppe, |
| Het | einen fünf- oder sechsgliedrigen heteroaromatischen Rest mit 1 bis 3 N-, O- und/oder S-Atomen, der auch mit einem Benzol- oder Pyridinring kondensiert sein kann, |
| Hal | F, Cl, Br oder I, |
| k | 0, 1, 2, 3 oder 4, |
| m und n | jeweils 1, 2 oder 3 und |
| t | 1, 2, 3 oder 4 bedeuten, |

sowie ihre Salze.

**2.** 1,2-Dihydro-1-(2'-(4,5-dihydro-5-oxo-1,2,4-oxadiazol-3-yl)-biphenylyl-4-methyl)-2-oxo-3-N-methyl-N-methylsulfonyl-aminomethyl-6-butyl-pyridin und dessen K-Salz.

**3.** Verfahren zur Herstellung von 1,2-Dihydro-2-oxopyridinen der Formel I nach Anspruch 1 sowie ihrer Salze, dadurch gekennzeichnet, daß man

(a) eine Verbindung der Formel II

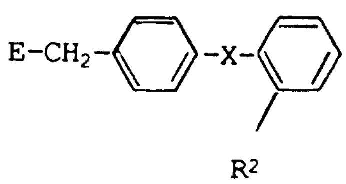

II

worin

E Cl, Br, I oder eine freie oder reaktionsfähig funktionell abgewandelte OH-Gruppe bedeutet und

$R^2$ die in Anspruch 1 angegebene Bedeutung hat,

mit einer Verbindung der Formel III

H-R III

worin

R die in Anspruch 1 angegebene Bedeutung hat,

umsetzt

oder

(b) eine Verbindung der Formel I aus einem ihrer funktionellen Derivate durch Behandeln mit einem solvolysierenden oder hydrogenolysierenden Mittel in Freiheit setzt,

(c) zur Herstellung einer Verbindung der Formel I, worin $R^2$-$C(NH_2)$=NOH bedeutet, eine Verbindung, die der Formel I entspricht, aber an Stelle des Restes $R^2$ eine CN-Gruppe trägt, mit Hydroxylamin umsetzt oder

(d) zur Herstellung einer Verbindung der Formel I, worin $R^2$-$SO_2NH$-$COOR^5$, -$SO_2NH$-$COR^5$, -$SO_2NH$-$SO_2R^5$ oder -$SO_2NH$-$CONR^5R^6$ bedeutet, eine Verbindung, die der Formel I entspricht, aber an Stelle des Restes $R^2$ eine -$SO_2NH_2$-Gruppe trägt, mit einer Verbindung der Formel E-$COOR^5$, E-$COR^5$, E-$SO_2R^5$, E-$CONR^5R^6$ oder O=C=$NR^5$ umsetzt

und/oder daß man in einer Verbindung der Formel I einen oder mehrere Rest(e) R und/oder $R^2$, in einen oder mehrere andere Reste R und/oder $R^2$ umwandelt und/oder eine Base oder Säure der Formel I in eines ihrer Salze umwandelt.

**4.** Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- und Hilfsstoff in eine geeignete Dosierungsform bringt.

**5.** Pharmazeutische Zubereitung, gekennzeichnet durch einen Gehalt an mindestens einer Verbindung der Formel I nach Anspruch 1 und/oder einem ihrer physiologisch unbedenklichen Salze.

**6.** Verbindung der Formel I nach Anspruch 1 und ihre physiologisch unbedenklichen Salze zur Bekämpfung von Krankheiten.

**7.** Verwendung von Verbindungen der Formel I nach Anspruch 1 und/oder ihrer physiologisch unbedenklichen Salze zur Herstellung eines Arzneimittels.

**8.** Verwendung von Verbindungen der Formel I nach Anspruch 1 und/oder ihrer physiologisch unbedenklichen Salze bei der Bekämpfung von Krankheiten.